(19) Europäisches Patentamt
European Patent Office
Office européen des brevets

(11) **EP 1 947 952 B1**

(12) **EUROPÄISCHE PATENTSCHRIFT**

(45) Veröffentlichungstag und Bekanntmachung des Hinweises auf die Patenterteilung:
**01.08.2012 Patentblatt 2012/31**

(21) Anmeldenummer: **06818268.2**

(22) Anmeldetag: **20.10.2006**

(51) Int Cl.:
**B01F 15/00** (2006.01)     **A22C 5/00** (2006.01)
**A22C 11/02** (2006.01)     **G01N 33/12** (2006.01)
**B01F 3/18** (2006.01)

(86) Internationale Anmeldenummer:
**PCT/EP2006/010138**

(87) Internationale Veröffentlichungsnummer:
**WO 2007/051533 (10.05.2007 Gazette 2007/19)**

(54) **Vorrichtung und Verfahren zur Fettanalyse**

Device and process for fat analysis

Dispositif et procédé d'analyse des graisses

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **07.11.2005 DE 102005053348**

(43) Veröffentlichungstag der Anmeldung:
**30.07.2008 Patentblatt 2008/31**

(73) Patentinhaber: **CFS Germany GmbH
35216 Biedenkopf-Wallau (DE)**

(72) Erfinder:
• **HOFFMANN, Hartmut
57334 Bad Laasphe (DE)**
• **LINN, Stefan
65594 Runkel (DE)**

(74) Vertreter: **Wolff, Felix et al
Kutzenberger & Wolff
Anwaltssozietät
Theodor-Heuss-Ring 23
50668 Köln (DE)**

(56) Entgegenhaltungen:
**WO-A-83/03673          WO-A2-02/052257
WO-A2-2004/004468     US-A- 4 171 164**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 947 952 B1

**Beschreibung**

Die vorliegende Erfindung betrifft eine Fleischverarbeitungsvorrichtung mit mindestens einem Zerkleinerungsaggregat zum Zerkleinem und/oder Mischen von frischem und/oder gefrorenem Fleisch und mit mindestens einem Fördermittel, auf dem das Fleisch förderbar ist und das In Verarbeitungsrichtung hinter dem Zendeinerungsaggregat angeordnet ist, die eine Fettanalysevorrichtung zur Bestimmung des Fettgehaltes, der Dichte Im Fleisch und/oder des BEFFE (Bindegewebeelwelßfreies Fleischeiweiß) aufweist, sowie Verfahren zur Fettanalyse.

Fleischpmdukte dürfen oder sollen einen bestimmten Fettgehalt nicht überschmiten, daher spielt eine genaue Fettanalyse von Fleisch und eine exakte Einstellung von bestimmten Fettgehalten bei Fleischprodukten eine Immer größere Rolle. Vorzugsweise erfolgt die Analyse des Fettgehalts des Fleisches während der Verarbeitung in einer Fleischverarbeitungsvorrichtung. Es gibt deshalb eine Reihe von Vorschlägen, wie diese Messungen erfolgen sollten, die jedoch alle den Nachteil haben, dass sie sehr aufwendig und/oder teilweise ungenau sind, bzw. erst sehr spät Im Prozess erfolgen Fettanalysevorrichtungen sind beispielweise aus der WO/02/052257A2 bekannt.

Die Fettanalyse kann bereits kontinuierlich erfolgen, indem beispielsweise der Fettgehalt von Fleisch auf einem Förderband mit einem Sensor bestimmt wird. Diese Messungen haben jedoch den Nachteil, dass der Massestrom des Fleisches absolut konstant sein muss.

Es stellt sich deshalb die Aufgabe, eine Vorrichtung und ein Verfahren zur kontinuierlichen Bestimmung des Fettgehalts von Fleisch zur Verfügung zu stellen. die die Nachteile des Standes der Technik nicht aufweisen.

Die Aufgabe wird erfindungsgemäß durch eine Fleischverarbeitungsvorrichtung gemäß Patentanspruch 1 gelöst.

**BESTÄTIGUNGSKOPIE**

Mit der erfindungsgemäßen Fleischverarbeitungsvorrichtung kann der Feftgehalt von Frischflelsch, gefrorenem Fleisch und/oder deren Mischung analysiert werden. Es war für den Fachmann überaus erslaunlich und nicht zu erwarten, dass es mit der erfindungsgemäßen Fleischverarbeitungsvorrichtung gelingt, den Fettgehalt des zu verarbeitenden Fleischproduktes sehr genau zu bestimmen. Die erfindungsgemäße Vorrichtung hat den Vorteil, das der Fettgehalt von Fleisch kontinuierlich bestimmt werden kann, wobei der Massestrom des Fleisches ebenfalls kontinuierlich oder zumindest semikontinuierlich durch die Wiegevorrichtung bestimmbar ist und nicht konstant sein muss. Die Vorrichtung ist einfach herzustellen und einfach in einen Fleischherstellungsprozess zu integrieren. Insbesondere ist es möglich die Fettanalysevorrichtung und/oder die Wiegevorrichtung nachträglich in bestehenden Fleischverarbeitungslinien nachzurüsten. Die Messgenauigkeit beträgt ca. $\pm$ 1% bezogen auf die Standardabweichung.

Eine Fleischverarbeitungsvorrichtung im Sinne der Erfindung ist jede dem Fachmann bekannte Maschine zur Fleischverarbeitung, mit der Fleisch zerkleinert und/oder gemischt wird. Vorzugsweise ist die Fleischverarbeitungsvorrichtung jedoch ein Mischer oder eine Zerkleinerungsmaschine, insbesondere ein Wolf, oder eine Kombination daraus.

Als Fördermittel kommen beliebige zur Lebensmittelverarbeitung geeignete Fördergerät in Betracht. In einer bevorzugten Ausführungsform ist das Fördermittel ein Förderband, vorzugsweise ein motorgetriebenes Kunststofffför-derband, auf dem das Fleisch beispielsweise von dem Zeddeinemngsaggregat, vorzugsweise einem Wolf, abtransportiert wird, beispielsweise in einen Behälter oder Mischer.

Die Wiegevotrichtung in der erfindungsgemäßen Vorrichtung ist vorzugsweise eine Bandwaage, die vorteilhafterweise leicht in das Förderband integrierbar ist. Die Wiegevorrichtung weist besonders bevorzugt eine Messstrecke auf. wobei mit der Wiegevorrichtung kontinuierlich ein momentanes Flächengewicht des Fleisches auf der Messstrecke bestimmbar ist.

Als Fettanalysemittel kommt jedes dem Fachmann geläufige Fettanalysemittel in Frage. Vorzugsweise weist das Fettanalysemittel jedoch eine Strahlenquelle, beispielsweise mit mehreren Energiestufen und einen Strahlendetektor auf. In einer besonders bevorzugten Ausruhrungsform ist die Strahlungsquelle eine Röntgenquelle und der Strahlungsdetektor ein Röntgendetektor. Ebenfalls bevorzugt ist als Strahlungsquelle eine Infrarotquelle und als Strahlungsdetektor ein Infrarotdetektor.

Bei der Fettanalyse mittels Röntgendetektor wird die Abschwächung des Röntgenstrahls vorzugsweise In einem Energiebereich zwischen 18 und 45 keV gemessen. Vorzugsweise beträgt der Messweg, insbesondere die durchstrahlte Fleischschicht 20 bis 300 mm, besonders bevorzugt 50 bis 100 mm, ganz besonders bevorzugt 50 - 70 mm. Die Berechnung des Fettgehaltes sowie die Steuerung der Röntgenquelle erfolgt durch einen Mikroprozessor oder Speicher-programmierbare Steuerung (SPS).

Ebenfalls bevorzugt erfolgt die Fettanalyse mit Near Infrared Transmisston (NIT).

Die Fettanalyse kann an jeder Stelle des Zerkleinerungsaggregats erfolgen, bei der eine Messstrecke zwischen der Strahlenquelle und dem Strahlendetektor zumindest zeitweise nicht von sich bewegenden Teilen, Insbesondere Metallteilen unterbrochen wird.

Das Zerkleinerungsaggregat weist vorzugsweise mindestens eine Förderaggregat auf, beilspielsweise eine Förderschnecke, wobei das Förderaggregat das Fleisch durch das Zerklelnerungsaggregat presst. Fleisch im Sinne der Erfindung ist Jedes Produkt, das Fleisch zumindest teilweise enthält. Die Fettanalyse kann beispielsweise im Bereich des Förderaggregat erfolgen.

Weiterhin bevorzugt weist das Zerkleinerungsaggregat zumindest einen Vorachneider auf, sowie weiterhin beispielsweise eine Lochscheibe, die mit einem Messer zusammenwirkt. In dem Vorschneider beziehungsweise in der Lochscheibe und/oder dem Messer wird das zu Fleisch zerkleinert und/oder vermischt. Vorzugsweise ist die Fettanalysevorrichtung in einem Vorschneider, einem sogenannten Messvorschneider angeordnet. Dieser kann, bezogen auf die Verarbeitungsrichtung des Fleisches hinter einem weiteren Vorschneider angeordnet sein. Dieser Messvorschnelder weist Ausnehmungen auf, an deren Kanten das Fleisch nach der Vorzerkleinerung in dem weiteren Vorschnelder vorzugsweise nicht weiter zerkleinert wird. Die Messstrecke der Fettanalysevorrichtung ist vorzugsweise In einer dieser Ausnehmungen angeordnet.

Der zusätzliche Vorschnelder vor dem Messvorschneider hat den Vorteil, dass ein Verschleiß an dem Vorschneider statt findet und an dem Messvorschneider dadurch zumindest vermindert ist. Durch die Vorzerkleinerung des Produktes In dem Vorschneider wird die Genauigkeit der Fettanalyse erhöht. Beispielsweise kann der Vorschneider auch mit einem zusätzlichen Messer zusammenwirken, das bezogen auf die Verarbeitungsrichtung des Fleisches vor dem Vorschneider angeordnet ist. Dieses zusätzliche Messer sorgt für einen sauberen Schnitt des Fleisches, so dass sich beispielsweise kein Bindegewebe im Bereich des Vorschneiders sammelt. Hierdurch wird verhindert, dass Fleisch mit beliebigem Fettgehalt für längere Zeit in der Messkammer verweilt, was sich ebenfalls positiv auf die Analysequalität auswirkt

Erfindungsgemäß weist die erfindungsgemäße Fleischverarbeitungsvorrichtung zusätzlich eine Geschwindigkeitsmessung auf, so dass neben dem momentanen Fettgehalt und dem momentanen Gewicht auch die momentane Geschwindigkeit des Fleisches bestimmt werden kann, wobei die Messung der Fördergeschwindigkeit nicht im Bereich der Fettanalysevorrichtung oder der Wiegevorrichtung erfolgen muss. Die Messung der momentanen Fördergeschwindigkeit des Fleisches erfolgt Vorzugsweise mit einem optischen Messverfahren und ebenfalls vorzugsweise nach dem Zerkleinern und/oder Mischen des Fleisches nahezu drucklos, also etwa bei Umgebungsdruck. Das optische Messverfahren basiert beispielsweise auf einer Lichtquelle, wie einer Halogenlampe kombiniert mit einer CCD-Kamera.

Ebenfalls bevorzugt entspricht die Fördergeschwindigkeit des Fleisches der Bandgeschwindigkeit des Fördermittels, die vorzugsweise durch Zählen der Bandumläufe ermittelbar ist. Eine aufwändige Messung der Gesdiwindigkeit kann so vorteilhafterweise entfallen. Besonders bevorzugt arbeiten die Fettanalysevorrichtung und die Wiegevorrichtung kontinuierlich. Die Messungen sind vorzugsweise zeitabhängig oder bandumlaufabhängig auswertbar. Beilspielsweise erfolgt die Auswertung alle 1 bis 2 Sekunden oder alle 5 bis 10 cm Bandlänge des Fördermittels.

Ein weiterer Gegenstand der vorliegenden Erfindung ist ein Verfahren zur kontinuleilchen Bestimmung des Fettgehaftes von Fleisch gemäß Patentanspruch 12.

Der Fachmann versteht, dass das Fleisch, dessen Fettgehalt momentan gemessen wird erst zeltverzögert gewogen wird. Die Zeitverzögerung ist wiederum von dem Abstand der Messpunkte, sowie der Fördergeschwindigkeit abhängig, wobei der Abstand in der Regel konstant ist, während die Geschwindigkeit ebenfalls konstant oder vorgegeben sein kann, oder vorzugsweise gemessen werden kann.

Der zeitabhängige Massenstrom Ft wird gemäß der folgenden Formel:

$$Ft \ [g/s] = G \ [g/cm2] * b \ [cm] * v \ [cm/s]$$

berechnet, wobei:

G     das momentane Flächengewicht in der Messstrecke (5),
b     die Breite des Messstrecke (5) und
v     die Fördergeschwindigkeit des Fleisches ist.

Ebenfalls bevorzugt können die Messungen in Abhängigkeit des Bandumlaufs des Fördermittels erfolgen. In diesem Fall ist statt einer Zeitabhängigkeit des Massenstroms eine Abhängigkeit von den Bandumläufen gegeben.

Der auf die Bandumläufe des Fördermittels bezogene Massenstrom Fb in Gramm pro Umlauf wird gemäß der folgenden Formel

$$Fb \ [g/U] = G \ [g/cm2] * b \ [cm] * I \ [cm/U]$$

berechnet, wobei:

G     das momentane Flächengewicht in der Messstrecke (5),
b     die Breite des Messstrecke (5) und
l     die Bandlänge des Fördermittels (2) in cm pro Umlauf.

Besonders bevorzugt wird ein mittlerer Fettgehalt einer resultierenden Fleischmischung aus dem momentanen Fettgehalt f des Fleisches und dessen Massenstrom F errechnet wird, insbesondere indem die Produkte von momentanem Fettgehalt f und zugehörigem Massenstrom Ft oder Fb aufsummiert werden und durch den aufsummierten Massenstrom, geteilt werden, gemäß der Formel:

$$\text{Fettgehalt} = \Sigma\ Ft\ {}^* f\ /\ \Sigma\ Ft = \Sigma\ Fb\ {}^* f\ /\ \Sigma\ Fb.$$

Der Fachmann versteht dass die Summe Σ Ft durch Summation über die Zeit und die Summe Σ Fb durch Summation über die Bandumläufe des Fördermittels erfolgt. Dieses Verfahren hat den Vorteil, dass durch die laufende Berechnung des mittleren Fettgehalts einer Fleischmischung ein Zeitverzug vorteilhaft vermieden wird. Es müssen keine zusätzliche Vorrichtungen zur Verfügung gestellt werden, mit denen der mittlere Fettgehalt der Fleischmischung zwischenzeitlich oder nach Fertigstellung bestimmt wird. Der Fettgehalt kann sowohl in frischem als auch in gefrorenem Fleisch bestimmt werden. Durch das erfindungsgemäße Verfahren wird die Konsistenz des Fleisches nicht verändert.
Die Werte des Fettgehalts und die Gewichtswerte des Fleisches können über einen Zeitraum von beispielsweise 1 bis 10 Sekunden, vorzugsweise 2 bis 4 Sekunden oder entsprechenden Bandvorschub des Fördermittels gemittelt werden.
Mit dem erfindungsgemäßen Verfahren ist es insbesondere möglich, auch den Fettgehalt bei einer nicht konstanten Fördermenge zu messen. Mit dem erfindungsgemäßen Verfahren ist es möglich, dem Massenstrom des untersuchten Fleisches den zugehörigen, momentanen Fettgehalt zuzuordnen und diese Daten beispielsweise an einen Zentralrechner zu überspielen, so dass auf dem zu verkaufenden Produkt zum Beispiel ein maximaler Fettgehalt des Produkts angegeben werden kann.
Im folgenden wird die Erfindung anhand der **Figuren 1-3** erklärt. Diese Erklärungen gelten sowohl für die erfindungsgemäße Vorrichtung, als auch für die erfindungsgemäßen Verfahren. Sie sind lediglich beispielhaft und schränken den allgemeinen Erfindungsgedanken nicht ein.

**Figur 1** zeigt eine schematische Darstellung einer erfindungsgemäßen Fleischverarbeitungsvorrichtung.
**Figur 2** zeigt ein Ausführungsbeispiel eines Zerkleinerungsaggregats mit Fettanalysevorrichtung.
**Figur 3** zeigt die Anordnung der Fettanalysevorrichtung im Bereich eines Messvorschneiders im detail.

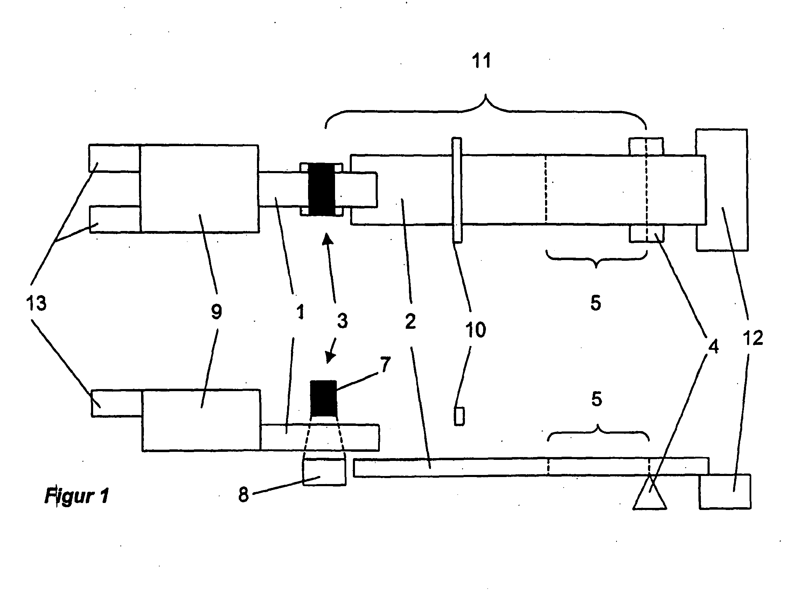

In der **Figur 1** ist die erfindungsgemäße Fleischverarbeitungsvorrichtung in zwei Ansichten schematisch dargestellt. Die Fleischverarbeitungsvorrichtung weist ein Zerkleinerungsaggregat 1, sowie ein Förderaggregat 9 auf, das im Sinne dieser Erfindung als Teil des Zerkleinerungsaggregats 1 definiert ist. Über wenigstens zwei Förderströme 13, die in der oberen Ansicht nebeneinander dargestellt sind, wird der Fleischverarbeitungsvorrichtung frisches und/oder gefrorenes Fleisch zugeführt. Dieses wird in dem Zerkleinerungsaggregat zerkleinert und/oder gemischt.
Das Fleisch der Förderströme 13 weist beispielsweise unterschiedliche Fettanteile auf. Durch eine Regulierung der Förderströme 13 lässt sich also ein momentaner Fettgehalt des Massenstroms durch die Fleischverarbeitungsvorrichtung beeinflussen und dadurch ein durchschnittlicher Fettgehalt in der resultierenden Fleischmischung 12 gezielt einstellen. Der Transport des zerkleinerten und/oder gemischten Fleisches von dem Zerkleinerungsaggregat 1 zu einem Behälter mit der Fleischmischung 12, erfolgt über ein Fördermittel 2, vorzugsweise ein Bandförderer. Mit einer Geschwindigkeitsmessung 10 wird kontinuierlich die Fördergeschwindigkeit des Fleisches gemessen.
Im Bereich des Zerkleinerungsaggregats 1 ist eine Fettanalysevorrichtung 3 angeordnet, die eine Strahlenquelle 8 und einen Strahlendetektor 7 aufweist, die am besten in der unteren Darstellung erkennbar sind. Beispielsweise handelt es sich um eine Röntgenquelle 7 und einen Röntgendetektor 8. Das Fleisch wird von dem Förderaggregat 9 durch das Zerkleinerungsaggregat 1 an der Röntgenquelle 8 vorbei gepresst und dabei von Röntgenstrahlen durchstrahlt. Der Röntgendetektor 7 misst die Absorption der Röntgenstrahlen durch das Fleisch. Anhand dieser Informationen kann auf den Fettgehalt des Fleisches geschlossen werden.
An dem Fördermittel 2 ist eine Wiegevorrichtung 4 angeordnet, vorzugsweise eine Bandwaage. Mit der Wiegevorrichtung 4 lässt sich das Gewicht des Fleisches bestimmen, welches sich auf einer Messstrecke 5 des Förderbands 2 befindet. Der Fachmann erkennt, dass aus diesen, kontinuierlich ermittelten Messwerten unter Berücksichtigung der Fördergeschwindigkeit ein Massenstrom des Fleischs durch die erfindungsgemäße Fleischverarbeitungsvorrichtung errechnet werden kann.

Die Fettanalysevorrichtung 3 und die Wiegevorrichtung 4 sind in einem Abstand 11 entlang der Förderstrecke voneinander beabstandet. Durch die räumliche Trennung der Vorrichtungen können einfachere Messgeräte verwendet werden. Die Integrierung in bestehende Verarbeitungslinien ist einfach und kostengünstig. Der Fachmann versteht, dass die Messwerte der Fettanalysevorrichtung 3 und der Wiegevorrichtung 4 einander zugeordnet werden müssen. Die Zuordnung ist abhängig von der Zeitspanne, die vergeht, während eine beliebig kleine Portion Fleisch, deren Fettgehalt gerade bestimmt wurde, zur Wiegevorrichtung 4 transportiert wird. Diese ist aus dem Abstand 11 und der Fördergeschwindigkeit leicht zu ermitteln. Vorzugsweise wird die Fördergeschwindigkeit durch die Geschwindigkeitsmessung 10 oder über die Bandumlaufgeschwindigkeit des Fördermittels 2 bestimmt.

Durch Aufsummierung der Produkte von Massenstromwerten und zugehörigen Fettgehalten und nachfolgendes dividieren durch den aufsummierten Massenstrom, also das aktuelle Gesamtgewicht der Fleischmischung 12, erhält man den aktuellen Fettgehalt der Fleischmischung 12.

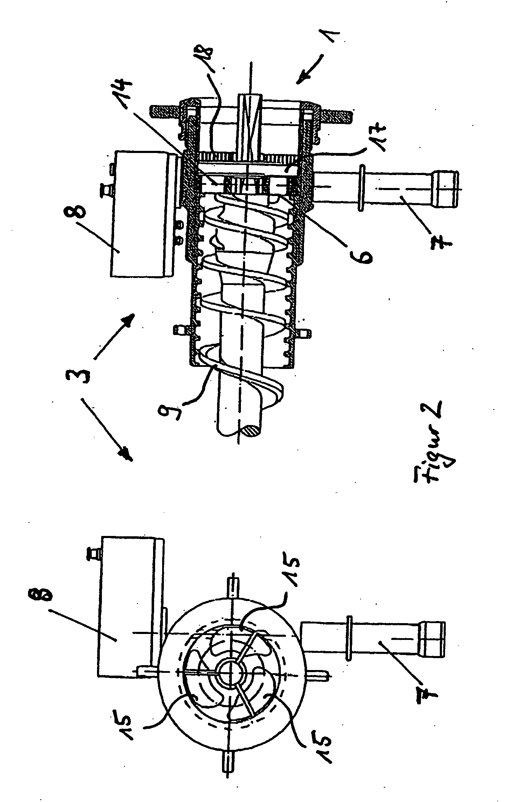

**Figur 2** zeigt eine Zerkleinerungsvorrichtung 1 mit einer Fettanalysevorrichtung 3 in zwei Ansichten. Ein Förderaggregat 9, hier eine Schnecke, fördert das Fleisch durch einen Schneidsatz der Zerkleinerungsvorrichtung 1. Der Schneidsatz besteht aus einem Vorschneider 6, einem nachgeordneten Messer 17 und einer Lochscheibe 18, wobei das Messer 17 mit der Lockscheibe 18 zusammenwirkt. Der Fachmann erkennt, dass vor dem Vorschneider 6, ein weiterer Vorschneider und ebenfalls noch ein weiteres Messer vorhanden sein kann. An dem Vorschneider 6 wäre der Verschleiß stark vermindert.

Im Bereich des Messvorschneiders 6 erfolgt die Messung der Fettanalysevorrichtung 3, die aus einer Strahlungsquelle 7 und einem Strahlungsdetektor 7 besteht. Von der Strahlungsquelle 8 wird ein Strahl, hier ein Röntgenstrahl 14 abgestrahlt, der wie aus dem linken Teil der Figur 2 ersichtlich ist, die Aussparung 15 in dem Messvorschneider 6 durchstrahlt und am Ende der Aussparung 15 von dem ' Strahlendetektor 7 empfangen und analysiert wird. Der Fachmann erkennt, dass auch andere Messprinzipien eingesetzt werden können. Bei Fettanalysevorrichtungen im Bereich von bewegten Teilen ist wichtig, dass die Messstrecke zum Zeitpunkt der Messung nicht unterbrochen ist bzw. wenn die Messstrecke zum Zeitpunkt der Messung unterbrochen sein sollte, diese Messwerte verworfen werden.

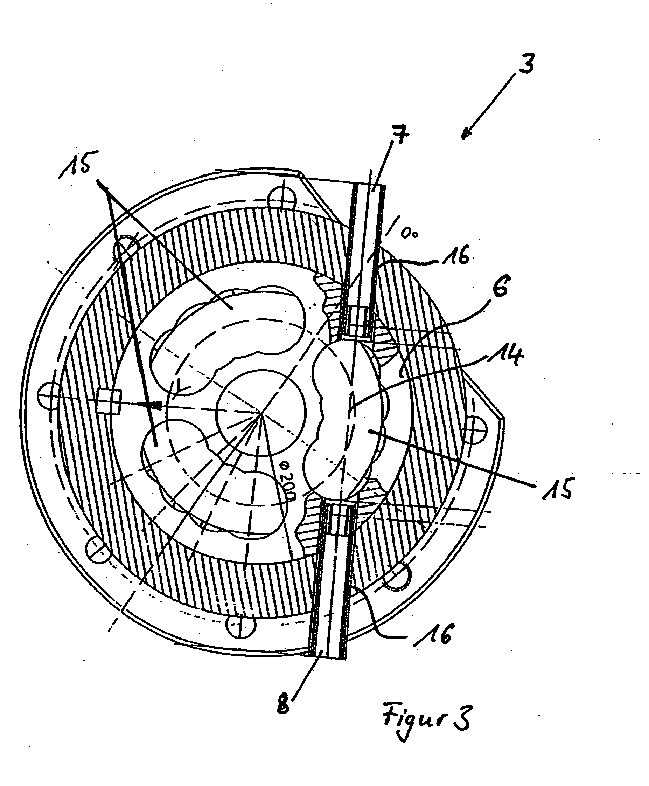

Die **Figur 3** zeigt eine Detaildarstellung des Messvorschneiders 6. Der Vorschneider 6 weist drei oder mehrere Aussparungen 15 auf, durch die das Fleisch gepresst wird. Im Bereich einer der Aussparungen 15 sind Bohrungen 16 angeordnet, in die die Strahlungsquelle 8 bzw. der Strahlungsdetektor 7 eingeführt werden. Die Strahlungsquelle 8 sendet einen Röntgenstrahl 14 aus, der das Fleisch, das sich in einer der Aussparungen 15 befindet, durchstrahlt. Aufgrund der unterschiedlichen Absorption der Strahlung 14 durch Fett- und Magerfleisch kann der Fettgehalt des sich in der Ausnehmung befindlichen Fleisches bestimmt werden.

**Bezugszeichen**

| | |
|---|---|
| 1 | Zerkleinerungsaggregat |
| 2 | Fördermittel |
| 3 | Fettanalysevorrichtung |
| 4 | Wiegevorrichtung |
| 5 | Messstrecke |
| 6 | Vorschneider |
| 7 | Strahlendetektor |
| 8 | Strahlenquelle |
| 9 | Förderaggregat |
| 10 | Geschwindigkeitsmessung |
| 11 | Abstand |
| 12 | Fleischmischung |
| 13 | Förderstrom |
| 14 | Messstrahl |
| 15 | Aussparung |
| 16 | Bohrung |
| 17 | Messer |
| 18 | Lochscheibe |

**Patentansprüche**

1. Fleischverarbeitungsvonichtung mit mindestens einem Zerkleinerungsaggregat (1) zum Zerkleinern und/oder Mischen von frischem und/oder gefrorenem Fleisch und mit mindestens einem Fördermittel (2), auf dem das Fleisch mit einer bestimmbaren Fördergeschwindigkeit förderbar ist und das in Verarbeitungsrichtung hinter dem Zerkleinerungsaggregat (1) angeordnet ist, wobei eine Fettanalysevorrichtung (3) zur kontinuierlichen Bestimmung eines Fettgehalts des Fleisches im Zerkleinerungsaggregat (1) angeordnet ist, **dadurch gekennzeichnet, dass** eine Wiegevorrichtung (4) an dem Fördermittel (2) angeordnet ist, wobei mit der Wiegevorrichtung das Gewicht des auf dem Fördermittel (2) geförderten Fleisches kontinuierlich bestimmbar ist und unter Einbeziehung eines Abstandes (11) zwischen der Wiegevorrichtung (4) und der Fettanalyseeinheit (3), sowie der Fördergeschwindigkeit des Fleisches ein momentaner Fettgehalt des Fleisches einem Massenstrom zuordenbar ist.

2. Fleischverarbeitungsvorrichtung nach Anspruch 1, **dadurch gekennzeichnet, dass** das Fördermittel (2) ein Förderband, vorzugsweise ein motorgetriebenes Kunststoffförderband ist.

3. Fleischverarbeitungsvorrichtung nach Anspruch 1 oder 2, **dadurch gekennzeichnet, dass** die Wiegevorrichtung (4) eine Bandwaage ist.

4. Fleischverarbeitungsvorrichtung nach einem der vorhergehenden Ansprüche, **dadurch gekennzeichnet, dass** die Wiegevorrichtung (4) eine Messstrecke (5) aufweist, wobei mit der Wiegevorrichtung (4) kontinuierlich ein momentanes Flächengewicht der Messstrecke (5) bestimmbar ist.

5. Fleischverarbeitungsvorrichtung nach einem der vorhergehenden Ansprüche, **dadurch gekennzeichnet, dass** die Fettanalysevorrichtung (3) eine Strahlenquelle (8) und einen Strahlendetektor (7) aufweist und dass die Fettanalysevorrichtung (3) vorzugsweise auf Röntgenstrahlung und/oder Near Infrared Transmission (NIT) basiert.

6. Fleischverarbeitungsvorrichtung nach einem der vorhergehenden Ansprüche, **dadurch gekennzeichnet, dass** das Zerkleinerungsaggregat (1) mindestens ein Förderaggregat (9) aufweist, wobei das Förderaggregat (9) das Fleisch durch das Zerkleinerungsaggregat (1) presst.

7. Fleischverarbeitungsvorrichtung nach einem der vorhergehenden Ansprüche, **dadurch gekennzeichnet, dass** das Zerkleinerungsaggregat (1) einen Vorschneider (6) aufweist und die Fettanalysevorrichtung (3) in dem Vorschneider (6) angeordnet ist.

8. Fleischverarbeitungsvorrichtung nach einem der vorhergehenden Ansprüche, **dadurch gekennzeichnet, dass** sie eine Geschwindigkeitsmessung (10) zur Bestimmung einer Fördergeschwindigkeit des Fleisches aufweist, wobei die Geschwindigkeitsmessung (10) vorzugsweise mit einem optischen Verfahren erfolgt.

9. Fleischverarbeitungsvorrichtung nach einem der vorhergehenden Ansprüche, **dadurch gekennzeichnet, dass** die Geschwindigkeitsmessung (10) nach dem Zerkleinern und/oder Mischen nahezu drucklos erfolgt.

10. Fleischverarbeitungsvorrichtung nach einem der vorhergehenden Ansprüche, **dadurch gekennzeichnet, dass** die Fördergeschwindigkeit des Fleisches der Bandgeschwindigkeit des Fördermittels entspricht, die vorzugsweise durch Zählen der Bandumläufe ermittelbar ist.

11. Fleischverarbeitungsvorrichtung nach einem der vorhergehenden Ansprüche, **dadurch gekennzeichnet, dass** die Fettanalysevorrichtung (3) und die Wiegevorrichtung (4) kontinuierlich arbeiten, wobei die Messungen vorzugsweise zeitabhängig oder bandumlaufabhängig auswertbar sind.

12. Verfahren zur kontinuierlichen Bestimmung des Fettgehaltes von Fleisch mit einer Vorrichtung gemäß einem der Ansprüche 1 bis 11, **dadurch gekennzeichnet, dass** mit der Fettanalysevorrichtung (3) kontinuierlich der Fettgehalt des Fleisches bestimmt wird und dass mit der Wiegevorrichtung (4) kontinuierlich das Gewicht des Fleisches bestimmt wird und dass unter Einbeziehung eines Abstands (11) zwischen der Wiegevorrichtung (4) und der Fettanalysevorrichtung (3), sowie der Fördergeschwindigkeit des Fleisches ein momentaner Fettgehalt einem Massenstrom des Fleisches zugeordnet wird.

**13.** Verfahren nach Anspruch 12, **dadurch gekennzeichnet, dass** der zeitliche Massenstrom Ft gemäß der folgenden Formel

$$Ft\ [g/s] = G\ [g/cm2] * b\ [cm] * v\ [cm/s]$$

berechnet wird, wobei:

G das momentane Flächengewicht in der Messstrecke (5),
b die Breite der Messstrecke (5) und
v die Fördergeschwindigkeit des Fleisches ist.

**14.** Verfahren nach Anspruch 12, **dadurch gekennzeichnet, dass** der auf die Bandumläufe des Fördermittels (2) bezogene Massenstrom Fb in Gramm pro Umlauf gemäß der folgenden Formel

$$Fb\ [g/U] = G\ [g/cm2] * b\ [cm] * l\ [cm/U]$$

berechnet wird, wobei:

G das momentane Flächengewicht in der Messstrecke (5),
b die Breite der Messstrecke (5) und
l die Bandlänge des Fördermittels (2) in cm pro Umlauf.

**15.** Verfahren nach einem der Ansprüche 12 bis 14, **dadurch gekennzeichnet, dass** ein mittlerer Fettgehalt einer resultierenden Fleischmischung aus dem momentanen Fettgehalt f des Fleisches und dessen Massenstrom Ft oder Fb errechnet wird, insbesondere indem die Produkte der momentanen Fettgehalte f und zugehörigen Massenströme F aufsummiert werden und durch den aufsummierten Massenstrom dividiert werden, gemäß der Formel:

$$Fettgehalt = \Sigma\ Ft * f\ /\ \Sigma\ Ft = \Sigma\ Fb * f\ /\ \Sigma\ Fb,$$

wobei die Summation Σ Ft über die Zeit und die Summation Σ Fb über die Umläufe des Fördermittels (2) erfolgt.

**Claims**

**1.** Meat processing device having at least one comminution unit (1) for comminuting and/or mixing fresh and/or frozen meat and having at least one conveying means (2), on which the meat can be conveyed at a determinable conveying speed and which is arranged after the comminution unit (1) in the processing direction, a fat analysis device (3) for the continuous determination of a fat content of the meat being arranged in the comminution unit (1), **characterized in that** a weighing device (4) is arranged on the conveying means (2), it being possible for the weight of the meat conveyed on the conveying means (2) to be determined continuously by the weighing device and, by incorporating a distance (11) between the weighing device (4) and the fat analysis unit (3) and also the conveying speed of the meat, it being possible to assign an instantaneous fat content of the meat to a mass flow.

**2.** Meat processing device according to Claim 1, **characterized in that** the conveying means (2) is a conveyor belt, preferably a motor-driven plastic conveyor belt.

**3.** Meat processing device according to Claim 1 or 2, **characterized in that** the weighing device (4) is a belt weigher.

**4.** Meat processing device according to one of the preceding claims, **characterized in that** the weighing device (4) has a measuring section (5), it being possible for an instantaneous weight per unit area of the measuring section

7

(5) to be determined continuously by the weighing device (4).

5. Meat processing device according to one of the preceding claims, **characterized in that** the fat analysis device (3) has a radiation source (8) and a radiation detector (7), and **in that** the fat analysis device (3) is preferably based on X-radiation and/or near infrared transmission (NIT).

6. Meat processing device according to one of the preceding claims, **characterized in that** the comminution unit (1) has at least one conveying unit (9), the conveying unit (9) forcing the meat through the comminution unit (1).

7. Meat processing device according to one of the preceding claims, **characterized in that** the comminution unit (1) has a pre-cutter (6) and the fat analysis device (3) is arranged in the pre-cutter (6).

8. Meat processing device according to one of the preceding claims, **characterized in that** it has a speed measurement (10) for determining a conveying speed of the meat, the speed measurement (10) preferably being carried out by an optical method.

9. Meat processing device according to one of the preceding claims, **characterized in that** the speed measurement (10) is carried out virtually without pressure after the comminution and/or mixing.

10. Meat processing device according to one of the preceding claims, **characterized in that** the conveying speed of the meat corresponds to the belt speed of the conveying means, which can preferably be determined by counting the belt revolutions.

11. Meat processing device according to one of the preceding claims, **characterized in that** the fat analysis device (3) and the weighing device (4) operate continuously, it preferably being possible for the measurements to be evaluated as a function of time or as a function of belt revolutions.

12. Process for the continuous determination of the fat content of meat with a device according to one of Claims 1 to 11, **characterized in that** the fat content of the meat is determined continuously with the fat analysis device (3) and **in that** the weight of the meat is determined continuously with the weighing device and **in that**, by incorporating a distance (11) between the weighing device (4) and the fat analysis device (3) and also the conveying speed of the meat, an instantaneous fat content is assigned to of a mass flow of the meat.

13. Process according to Claim 12, **characterized in that** the mass flow Ft over time is calculated in accordance with the following formula:

$$\text{Ft [g/s]} = \text{G [g/cm}^2\text{]} * \text{b [cm]} * \text{v [cm/s]}$$

where:

G is the instantaneous weight per unit area in the measuring section (5),
b is the width of the measuring section (5) and
v is the conveying speed of the meat.

14. Process according to Claim 12, **characterized in that** the mass flow Fb in gram per revolution, based on the belt revolutions of the conveying means (2), is calculated in accordance with the following formula

$$\text{Fb [g/rev]} = \text{G [g/cm}^2\text{]} * \text{b [cm]} * \text{l [cm/rev]}$$

where:

G is the instantaneous weight per unit area in the measuring section (5),

b is the width of the measuring section (5) and

l is the belt length of the conveying means (2) in cm per revolution.

15. Process according to one of Claims 12 to 14, **characterized in that** an average fat content of a resultant meat mixture is calculated from the instantaneous fat content f of the meat and its mass flow Ft of Fb, in particular by the products of instantaneous fat content f and associated mass flows F being summed and divided by the summed mass flow, in accordance with the formula:

$$\texttt{Fat content = ΣFt*f / ΣFt = ΣFb*f / ΣFb,}$$

the summation ΣFt being made over time and the summation ΣFb being made by summing over the revolutions of the conveying means (2).

## Revendications

1. Procédé de traitement de viande comprenant au moins une unité de hachage (1) pour hacher et/ou mélanger de la viande fraîche et/ou congelée et comprenant au moins un moyen de transport (2) sur lequel la viande peut être transportée à une vitesse de transport déterminable, et qui est disposé dans la direction de traitement derrière l'unité de hachage (1), un dispositif d'analyse des graisses (3) prévu pour déterminer en continu une teneur en graisses de la viande étant disposé dans l'unité de hachage (1), **caractérisé en ce qu'**un dispositif de pesée (4) est disposé sur le moyen de transport (2), le poids de la viande transportée sur le moyen de transport (2) pouvant être déterminé en continu avec le dispositif de pesée et en tenant compte d'une distance (11) entre le dispositif de pesée (4) et l'unité d'analyse des graisses (3), ainsi que de la vitesse de transport de la viande, une teneur en graisses instantanée de la viande pouvant être affectée à un flux massique.

2. Dispositif de traitement de viande selon la revendication 1, **caractérisé en ce que** le moyen de transport (2) est une bande transporteuse, de préférence une bande transporteuse synthétique entraînée par un moteur.

3. Dispositif de traitement de viande selon la revendication 1 ou 2, **caractérisé en ce que** le dispositif de pesée (4) est une bascule à bande.

4. Dispositif de traitement de viande selon l'une quelconque des revendications précédentes, **caractérisé en ce que** le dispositif de pesée (4) présente une section de mesure (5), un poids surfacique instantané de la section de mesure (5) pouvant être déterminé avec le dispositif de pesée (4).

5. Dispositif de traitement de viande selon l'une quelconque des revendications précédentes, **caractérisé en ce que** le dispositif d'analyse des graisses (3) présente une source de rayonnement (8) et un détecteur de rayonnement (7) et **en ce que** le dispositif d'analyse des graisses (3) est basé de préférence sur un rayonnement de rayons X et/ou sur une transmission infrarouge proche (NIT).

6. Dispositif de traitement de viande selon l'une quelconque des revendications précédentes, **caractérisé en ce que** l'unité de hachage (1) présente au moins une unité de transport (9), l'unité de transport (9) pressant la viande à travers l'unité de hachage (1).

7. Dispositif de traitement de viande selon l'une quelconque des revendications précédentes, **caractérisé en ce que** l'unité de hachage (1) présente un dispositif de coupe préliminaire (6) et le dispositif d'analyse des graisses (3) est disposé dans le dispositif de coupe préliminaire (6).

8. Dispositif de traitement de viande selon l'une quelconque des revendications précédentes, **caractérisé en ce qu'**il présente une mesure de vitesse (10) pour déterminer une vitesse de transport de la viande, la mesure de vitesse (10) s'effectuant de préférence avec un procédé optique.

9. Dispositif de traitement de viande selon l'une quelconque des revendications précédentes, **caractérisé en ce que** la mesure de vitesse (10) s'effectue après le hachage et/ou le mélange pratiquement sans pression.

**10.** Dispositif de traitement de viande selon l'une quelconque des revendications précédentes, **caractérisé en ce que** la vitesse de transport de la viande correspond à la vitesse de bande du moyen de transport, qui peut être déterminée de préférence par comptage des tours de bande.

**11.** Dispositif de traitement de viande selon l'une quelconque des revendications précédentes, **caractérisé en ce que** le dispositif d'analyse des graisses (3) et le dispositif de pesée (4) fonctionnent en continu, les mesures pouvant être analysées de préférence en fonction du temps ou en fonction des tours de bande.

**12.** Procédé pour déterminer en continu la teneur en graisses de viande avec un dispositif selon l'une quelconque des revendications 1 à 11, **caractérisé en ce que** la teneur en graisses de la viande est déterminée en continu avec le dispositif d'analyse des graisses (3) et **en ce que** le poids de la viande est déterminé en continu avec le dispositif de pesée (4) et **en ce qu'**en tenant compte d'une distance (11) entre le dispositif de pesée (4) et le dispositif d'analyse des graisses (3), ainsi que de la vitesse de transport de la viande, une teneur en graisses instantanée est associée à un flux massique de la viande.

**13.** Procédé selon la revendication 12, **caractérisé en ce que** le flux massique dans le temps Ft est calculé selon la formule suivante :

$$\texttt{Ft [g/s] = G [g/cm2] * b[cm]*v[cm/s]}$$

G étant le poids surfacique instantané dans la section de mesure (5),
b étant la largeur de la section de mesure (5) et
v étant la vitesse de transport de la viande.

**14.** Procédé selon la revendication 12, **caractérisé en ce que** le flux massique Fb en gramme par tour rapporté aux tours de bande du moyen de transport (2) est calculé selon la formule suivante :

$$\texttt{Fb [g/U] = G [g/cm2] * b [cm] * I [cm/U]}$$

G étant le poids surfacique instantané dans la section de mesure (5),
b étant la largeur de la section de mesure (5) et
I étant la longueur de bande du moyen de transport (2) en cm par tour.

**15.** Procédé selon l'une quelconque des revendications 12 à 14, **caractérisé en ce qu'**une teneur en graisses moyenne d'un mélange de viande résultant est obtenue à partir de la teneur en graisses instantanée f de la viande et de son flux massique Ft ou Fb, en particulier en additionnant les produits des teneurs en graisses instantanées f et les flux massiques associés F et en divisant par le flux massique additionné, selon la formule :

$$\texttt{teneur en graisses = Σ Ft * f / Σ Ft = Σ Fb * f/Σ Fb}$$

la somme Σ Ft s'effectuant en fonction du temps et la somme Σ Fb s'effectuant en fonction des tours du moyen de transport (2).

Figur 1

Figur 2

Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 02052257 A2